# EUROPEAN PATENT SPECIFICATION

(11) **EP 2 211 726 B1**
(45) Date of publication and mention of the grant of the patent: **12.12.2012**
(21) Application number: 07834578.2
(22) Date of filing: 16.10.2007
(51) Int. Cl.: A61B 17/08, A61B 19/08, A61F 13/00

(54) **DEVICE FOR ADHERING TO THE SKIN OF A PATIENT**
VORRICHTUNG ZUR HAFTBEFESTIGUNG AUF DER HAUT EINES PATIENTEN
DISPOSITIF CONÇU POUR ADHÉRER À LA PEAU D'UN PATIENT

(43) Date of publication of application: 04.08.2010
(73) Proprietor: Close-it B.V., 3763 LZ Soest (NL)
(72) Inventor: JEEKEL, Johannes, NL-3062 Rotterdam (NL); BATTERINK, Henri, NL-3828 SB Hoogland (NL)
(74) Representative: De Grave-Wolterink, Isabelle
(86) International application number: PCT/NL2007/000262
(87) International publication number: WO 2009/051472

(56) References cited:
- US-A- 4 038 989
- US-A- 5 197 493
- US-A- 5 263 970

## Description

The present invention relates to a device for adhering to the skin of a patient, suitable to make a skin incision there through, the device comprising means for closure of the incision wound.

The said wound can also be a larger surgical wound. In particular, the device is suitable for adhering to the skin at the location the incision or excision is to be made; where after the incision or excision is made through the device in the skin at the envisaged location.

Such devices are known in the art. E.g. WO 88/08690 describes a wound closure device having a backing layer having an adhesive surface for adhering to the skin and comprising, on the opposed surface, adhesive coated strips that are arranged in an S or Z configuration. These strips are arranged in two parallel arrays, which arrays are distanced from one and another. The incision is made through the baking layer between both arrays, after which the skin can be closed by folding the strips and adhere these onto the backing layer, extending over the location of the incision. The device according to WO 88/08690 may provide for complete coverage of the incision by the strips, which may result in equal closing forces along the edges of the wound, which is a significant improvement over wound closure by stitching. Stitching namely results in high closing forces at the site of the stitches, however significant lower closure forces between the stitches are achieved. The device according to WO 88/08690 has an important disadvantage in that the backing layer remains adhered to the skin, also at the site of the skin edges of the incision. The edges of the incision wound are therefore also covered by the backing layer. The wound edges are brought together by abutting the edges of the incised backing layer to one another by using the strips. There is no direct control of the skin below the backing layer, which may result in suboptimal closing and in suboptimal scar formation.

Such a device is further not suitable for performing excisions from the skin, e.g. when a small melanoma has to be excised. In that case, the excision would also be made in the basic layer, and re-abutting the edges of the basic layer is not well possible, as the excised portion of the layer is missing. The excision wound cannot be closed conveniently.

The same is true in the devices, as described in US 5,336,219, US 6,007,564 and EP 1.675.521. These devices all have a basic layer as described above, where through the incision is to be made.

The same problem is encountered in the device of US 4,531,521, wherein two separate backing layers, which may be held together by a removable cover layer, is placed onto the skin, wherein those layers abut one another. The incision is made precisely between these backing layers. After the incision is made, the wound can be closed by bringing the backing layers back together again. Again, direct contact with the skin surrounding the wound is not possible. Control of wound closing is only by re-abutting the backing layers to one another.

US 4,222,383 describes a closing device, comprising a backing layer to be adhered to the skin, which backing layer comprises two arrays of closing strips, which arrays are arranged at a distance and parallel to one another Between these arrays, the backing layer is however not adhered to the skin. At this location the incision is to be made, where after the wound is closed by connecting opposingly arranged thin closing strips to one another These strips are not adhered to the skin or to the basic layer Although the device according to US 4,222,383 may avoid stitching or stapling the wound, the closing effect will be similar of that of stitching as in each of the arrays, the closing strips are located at a distance from one another. As the wound is only closed at the location of the strips, a high local closing force is obtained at the location of the connected strips, whereas the closing force is significantly lower between the strips

US2004/0204740 and WO03/053296 describe devices for closing skin wounds without sutures, comprising two separate elongated base strips, each comprising bridging elements. The base strips are to be adhered to the skin adjacent to the skin wound after the incision is made. Then, base strips are connected to one another, therewith closing the wound, by adhering the bridging elements of the one base strip to the other base strip These devices are not suitable to make an incision there through, and the bridging elements are not suitable to adhere to the skin, only to the opposing base strip, so that the base strips must be located at the edges of the incision wound for skin closure.

The device according to the present invention envisages to improve at least one of the above disadvantages, and this is achieved by the device according to the present invention, in particular by the provision of one or more basic layers that do not abut one another at the location of the wound. In contrast, an open skin area is defined by the basic layer(s), which area is covered by an adhesive surface of a covering sheet Further, adhesive strips are provided that are suitable to be adhered to the skin at both sides of the incision, therewith closing the skin in a highly controlled manner The device allows an excision or incision to be made in a convenient manner, and enabling excision wounds to be conveniently closed as well.

The invention provides a device according to the main claim Further embodiments are described in the dependent claims.

US 4,038,989 discloses a therapeutic device for skin lesion in which adhesively coated flexible strips are stuck to the skin on opposed sides of the lesion. Flexible strips are stuck to the skin in a flat condition after a mark has been made on the skin, extending along the gap between the strips. Flaps are joined to the flexible strips by panels. A removable strip is provided on the flaps, for aligning the flaps to avoid lateral displacement of the skin. The flaps have an adhesive coating on their surfaces next to the removable strip. Hence, the removable strip in is not provided with an adhesive, and thus is the removable strip between the flaps not stuck to the skin. An incision is made along the marking by means of knife cutting through the removable strip into the skin. After the operation, the removable strip is peeled away and the flaps are pressed together to draw the panels closer to one another and close the gap between them, thus closing the skin over the lesion made by the incision.

US 5,263,970 discloses a surgical dressing for closing a wound. The surgical dressing includes a pair of strips being placed on opposed sides of the wound. Manipulating means interconnect the strips, the manipulating means being operable to draw the strips towards each other to close the wound. The manipulating means are integral with the strips and comprise a coating for bonding with the skin of a person.

The invention will now be further illustrated by way of example only, by reference to the following figures, which are however in no sense intended to limit the scope of the appended claims.
Figures 1 and 2A show a first and a more preferred second embodiment of the device according to the invention respectively, before assembly of cover layer, sheet members, bridging members, bridge covering members and sheets of backing material,
Figure 2B shows the assembly of the components of figure 2A,
Figure 3 is a top view of an assembly of a first and second sheet member, spacing members and bridging members, made of one piece, wherein the bridging members are folded backward onto the respective sheet members,
Figure 4 is an overview of the assembly of figure 3, wherein the bridging members are each covered by a bridge covering member,
Figure 5 is an upper view on an assembly as in figure 3, wherein the bridging members are closed,
Figure 6 is a cross section through line X of figure 4,
Figure 7 is a cross section of figure 6, wherein the assembly now comprises backing paper and a covering sheet,
Figure 8 is a cross section of another embodiment of an assembly of a bridging member, sheet member and bridge covering member,
Figure 9 is a cross section of an arrangement of 2 backing papers for the device according to the present invention,
Figure 10 is an arrangement of an assembly of 3 backing papers for the device according to the present invention,
Figure 11 is a schematic overview of different embodiments of the backing paper,
Figure 12 is an overview of different shapes of bridging members,
Figure 13 is an overview of different sheet members,
Figure 14 is an overview of different embodiments of different arrangements of bridging members and bridge covering members,
Figure 15 shows different embodiments of covering sheets,
Figure 16 shows different examples of alignment markers, according to a preferred embodiment of the present invention,
Figure 17A-N shows a cartoon of the use of the device wherein the device is according to the present invention for making and closing an incision wound in skin.

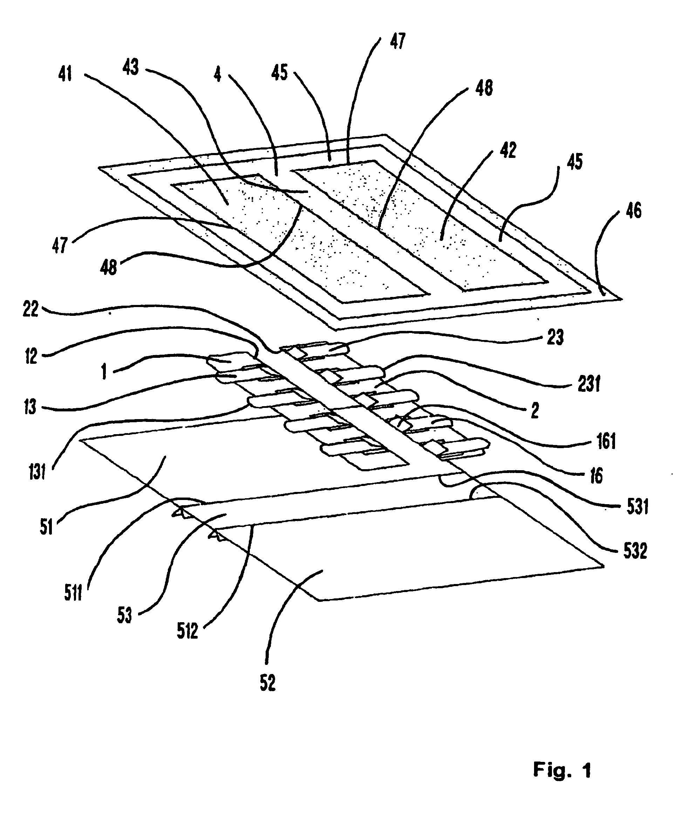

In figure 1, a first sheet member 1 and a second sheet member 2 each having a first surface (facing down), and a second surface (facing up) are positioned opposite to one another. The first sheet member 1 has a first opposing edge 12, whereas the second sheet member 2 has a second opposing edge 22. The first and second opposing edges oppose one another, therewith defining a bridge distance there between. The bridge distance is preferably at least 2 cm, leaving about 1 cm of surrounding skin of the wound, more preferably at least 3 cm, , leaving about 1.5 cm of surrounding skin of the wound. Preferably, the said bridging distance does not exceed 10 cm, leaving about 5 cm of surrounding skin of the wound, more preferably, the said bridging distance does not exceed 8 cm, leaving about 4 cm of surrounding skin of the wound. In the present case, both first and second opposing edges 12, 22 are straight and run parallel to one another. However, these edges can also be curved, so that the bridging distance can vary accordingly. However, straight opposing edges are preferred. The first surface of both the first and second sheet members 1, 2 comprise a first adhesive which is suitable to adhere the respective sheet member to the skin. The skilled person will be aware of suitable materials for both the adhesive and the sheet members. Preferably, the sheet member is air permeable, and is preferably a polyurethane foil having a thickness, preferably varying from 20 - 60 µm, more preferably of 25 - 50 µm, most preferably about 35 µm. With 'about' is meant that the thickness can vary 3 µm or less from the said value, i.e. being between 32 and 38 µm. The first adhesive should preferably ensure a good adherence over at least ten days, should preferably be removable without damaging the skin and should preferably also have sufficient tack upon contact with the skin preferred examples of such adhesives are hotmelt adhesives based on polyolefine, as are known to the skilled person, who will be aware of suitable alternatives . The length of opposing edges 12 and 22 define, together with the bridging distance there between, an open skin area, in which area the incision in the skin, or excision from the skin, should take place, once the sheet members are placed on the envisaged location on the skin.

The first and second sheet members 1, 2 comprise at their first and second opposing edges 12, 22, respectively, a plurality of bridging members 13, 23, four on the first sheet member 1 and five on the second sheet member 2. It is to be noted that it is preferred to design both first and second sheet members 1,2 in an identical fashion, i.e. having the same number of bridging members 13, 23. Each bridging member 13, 23 comprises a strip of sheet material, having a first and second surface, and the bridging members are folded backward to the first and second sheet members respectively. The sheet material of the bridging members have a first surface facing up when the bridging members are folded backward, and a second surface facing down when the bridging members are folded backward, like in figure 1. The first surface comprises a second adhesive, which is suitable to adhere the respective bridging member to the skin upon unfolding, and optionally also to the second surface to the opposing sheet member. Again this sheet material is preferably air permeable, and preferably a polyurethane foil, more preferably of the above identified specifications.

On top of the sheet members, a covering sheet 4 is positioned, comprising a first part 41, covering the first sheet member 1, a second part 42, covering the second sheet member 2, and a third part 43 covering the open skin area 3. Further, the covering sheet comprises in the embodiment shown, a circumfering portion 45, circumfering the first, second and third portions of the covering sheet. The covering sheet also comprises a first surface, facing down to the second surface of the first and second sheet members and to the open skin area, and a second surface, facing up. The first surface of the covering sheet 4 comprises a third adhesive at the third portion 43, and a fourth adhesive at the circumfering portion 45. Said third and fourth adhesive are also suitable to be releasably adhered to the skin, and are preferably the same adhesive. The cover sheet preferably comprises an additional edge portion 46 along the perimeter of the circumfering portion 45. Said additional edge portion can be made of a sheet material of more stiffness and/or strength than the rest of the covering sheet to confer additional strength to the device to improve the handling properties thereof. A suitable material is e.g. polypropylene, whereas the preferred material for the sheet member is polyurethane foil. Said additional edge portion can also e.g. be of a different colour than the rest of the covering sheet, to improve handling of the device.

The covering sheet preferably comprises first and/or second visual indication means 47 and 48, respectively, e.g. in the form of a coloured line. The first visual indication means indicate the location of the first and second sheet members, whereas the second visual indication means indicate the location of the open skin access area. In the present embodiment both first and second visual indication means are combined in the form of a box. These visual indication means can have any form, as long as the respective locations are indicated therewith. For example, the box, enclosed by the lines 47, 48 can be hatched. The visual indication means help the medical staff in positioning the device onto the skin of the patient. Also, the physician will know where to make the incision without the risk of cutting through the sheet members.

Upon placing the device according to the invention on the skin of the patient, the device will adhere to the skin by the adhering first and second sheet members, the third portion of the sheet member and, if present, by the circumfering portion of the covering sheet. The covering sheet covers the sheet members and the bridging members located thereon.

Preferably, the covering sheet does not adhere to the sheet members, as the covering sheet is to be removed from the skin after the incision of excision is made, whereas the sheet members should remain adhered to the skin with the bridging members in back-folded position. To this end, the first surface of the covering sheet 4 is, covering the first and second sheet members 1, 2 (i.e. at the first and second portion 41, 42 of the covering sheet 4) preferably free of adhesive. However, the adhesive of the covering sheet 4 may, to this end, locally be shielded, e.g. by a piece of sheet material covering the adhesive. If the covering sheet would adhere to the sheet members and/or the bridging members, the sheet members could also release from the skin, or the bridging members could move from their back-folded position, or even be damaged upon removal of the covering sheet. However, the adhesive can be chosen such that the tack between the covering sheet and the sheet members and the bridging members is less than the tack of the sheet member and the skin. And in that case, also the bridging members should, in back folded position, adhere to the respective sheet member, which can be accomplished by providing a suitable fifth adhesive between the second surface of the bridging member and the second surface of the sheet member, at a location where these surfaces abut one another. The tack between these surfaces should again be more than the tack of the third adhesive of the covering sheet in order to ensure proper positioning of the bridging members upon removal of the covering sheet. However, the fifth adhesive must still allow manual release of the bridging member from the respective sheet member.

The first and second sheet members 1, 2 are positioned on sheets of backing material. The sheet of backing material comprises a first, second and third sheet of backing paper 51, 52 and 53 respectively. A suitable material for sheets of backing material is known to the skilled person. The material can e.g. be paper, preferably comprising a silicon coating, but any suitable sheet like backing material may be used. However, a silicon coated material, such as silicon paper, is preferred. In the assembled situation, the backing papers 51, 52 and 53 are releasably attached to the first surface of the first and second sheet members 1, 2. It is also releasably attached to the third portion 43 of the first surface of the covering sheet 4, which covers the open skin area. Also the edges 45 of the covering sheet 4 attach releasably to the backing paper. In the present case, the covering sheet comprises an additional edge of a more rigid material, e.g of polypropylene, to facilitate proper handling of the assembly and removal of the covering sheet. However, any suitable material can be used. The backing paper 51 comprises a first abutting edge 511, abutting a primary abutting edge 531 of the third sheet 53, whereas the second sheet 52 comprises a second abutting edge 521, abutting the third sheet via the secondary abutting edge 532 of the third sheet 53. The sheets 51, 52 and 53 all extend with a certain length beyond their abutting edges 511, 521, 531 and 532 respectively, in order to facilitate removal thereof.

Once assembled, the device according to the invention is preferably packed in a suitable sterilisation bag so that the device can be sterilized after production and/or before use.

In a simpler embodiment, the device comprises two sheets of backing material, wherein the third sheet is not present. In that case, abutting edge 511 of the first sheet 51 abuts the second abutting edge 521 of the second sheet 52. The abutting edges of the first, second and third sheets of backing paper preferably run parallel to one another and preferably perpendicular to the length axis of the open skin area, which in this case runs parallel to the first and second opposing edges 12 and 22 of the first and second sheet member.

In the embodiment shown in figure 1, the first and second sheet members 1, 2 are not connected to one another, so that the distance there between can be freely chosen.

It is of importance to keep both sheet members in the envisaged position upon removal of the backing paper, in particular, when there is no tack between the covering sheet and the first and second sheet members. In order to provide proper positioning of both sheet members, the backing paper should be removed carefully, to avoid undesired repositioning of the sheet members. By the use of multiple backing papers, this problem can be minimized, as the sheet members can remain properly positioned when only one of the two or three backing papers is removed. As will be shown below, the embodiment having three backing papers is preferred.

The bridging members are provided, on their first surface, with a bridge covering member 16, said bridge covering member having a first surface (facing down) covering the adhesive first surface of the bridging members. The bridge covering members 16 are releasably attached to the respective bridging members 13, 23 by the second adhesive of the bridging members. The bridge covering members 16 are of a suitable sheet like material, keeping the adhesive of the bridging member intact upon the removal of the bridge covering member 16. A suitable material for the bridge covering member 16 is e.g. silicon paper, but the skilled person is aware of any suitable material. Although not shown in the figure, protruding ends 131, 231 of first and second bridging members 13, 23 respectively, can be connected to the respective bridge covering member 16, which is folded backward on the first surface of the bridging member. In the present case, the length of the bridge covering member exceeds that of the respective bridging member, wherein the exceeding end 161 of the bridge covering member 16 is folded backward at the location of the opposing edge of the sheet member that comprises the said bridging member. By this, the exceeding end 161 of the bridge covering member 16 allows being held by hand, in order to pull the bridge covering member 16 from the bridging member 13, 23 and therewith lifting and unfolding the bridging member as will be shown below. It is important that the adhesive of the bridging member 13, 23 allows tearing off the bridge covering member 16, however it should have sufficient tack to remain secured on the skin, as explained above.

In a particular attractive embodiment (not shown) the exceeding end 161 of the bridge covering member extends beyond the end of the bridging member 131, 231 to facilitate be picked up by hand even more. In that case, the length of the bridge covering member exceeds twice that of the respective bridging member.

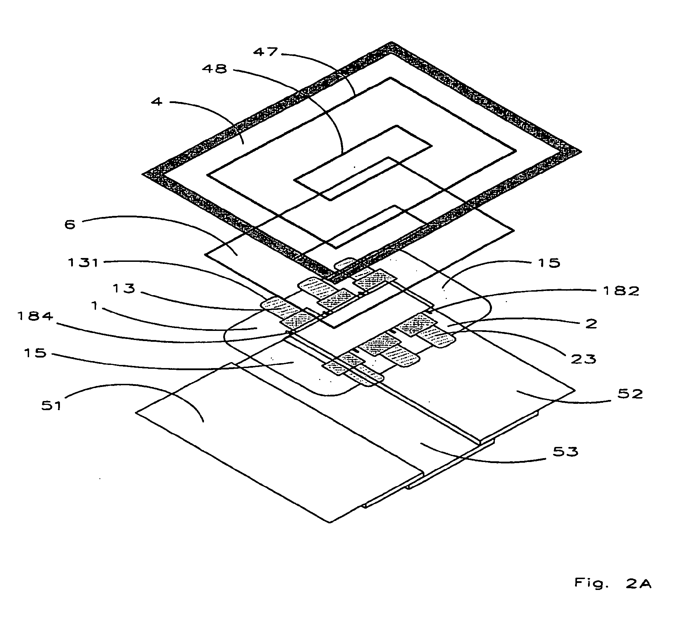

In figure 2A, a similar device as that of figure 1 is shown with a few additional advantages. Features that are the same with regard to the device as explained in figure 1, have the same reference numbers as in figure 1, or are not indicated. First of all, the first and second sheet members 1 and 2 are connected to one another by spacing members 15, which are in this case, of the same sheet material of sheet members 1 and 2. Although the spacing members can be made of separate pieces of material and be of different material than the sheet members, the sheet members and spacing members are preferably made of a single piece of sheet material. The spacing members also have a first surface, facing down, and a second surface, facing up. The first surface comprising an adhesive, suitable for releasably adhering the spacing member to the skin. In the present case, the first surface of the first and second sheet members and the first surface of the spacing members are of the same material and preferably, the adhesive of the first sheet members and of the spacing members are identical. The bridging members 13 and 23 are folded backwards onto the first and second sheet members 1, 2 respectively so that the complete second surface of the bridging member faces the second surface of the respective sheet member. However, the sheet member and the bridging member can be dimensioned such, that the protruding end 131 of the bridging member extends beyond the respective sheet member, so that only a portion of the second surface of the bridging member faces the second surface of the respective sheet member. Although the bridging members can be made of separate material and be connected to the first and second sheet members by the aid of a suitable adhesive, the first and second bridging members are preferably an integral part of the first and second sheet members 1, 2 respectively, so that the first sheet member and the first bridging members are made of a single piece of sheet and the second sheet member and bridging members as well. In the present case, both sheet members, the spacing members as well as the bridging members are made of a single piece of sheet material. The sheet members comprise alignment markers 182 and 184 on the second and first sheet member 2, 1 respectively. In the same axis of these alignment markers, a corresponding alignment marker is provided on the bridging members visible from the second surface thereof. As the bridging members are in a back folded position, the first surface of the bridging member faces up, which members are also covered by bridge covering members, as indicated in figure 1.

In this embodiment, the covering sheet 4 comprises first visual indication means 47, indicating the location of the first and second sheet members 1, 2 and spacing members 15, as well as second visual indication means 48, indicating the location of the open skin access area 3. These means can be designed e.g. as coloured lined in this embodiment. Again, these visual indication means help proper positioning of the device on the skin of the patient, and give a clear indication of the location of the open skin access area, wherein the incision is to be made. Again, any suitable visual indication means can be used, who are known to the skilled person. Coloured lines can be used, or the envisaged areas can be coloured completely, or hatched, etc. Further, in the assembly of this embodiment, the covering sheet adhered to a shielding member 6. By the presence of the shielding member, the first surface of the sheet member can be completely provided with adhesive, and at the location covering the first and second sheet members and the bridging members, the adhesive is shielded by the shielding member, avoiding undesired adherence of the covering sheet with the sheet members and bridging members.

The advantage of the presence of spacing members is e.g. that the position between the first and second sheet members is secured, therewith lowering the risk of undesired repositioning of any of the sheet members upon removal of the backing paper before positioning the device on the skin.

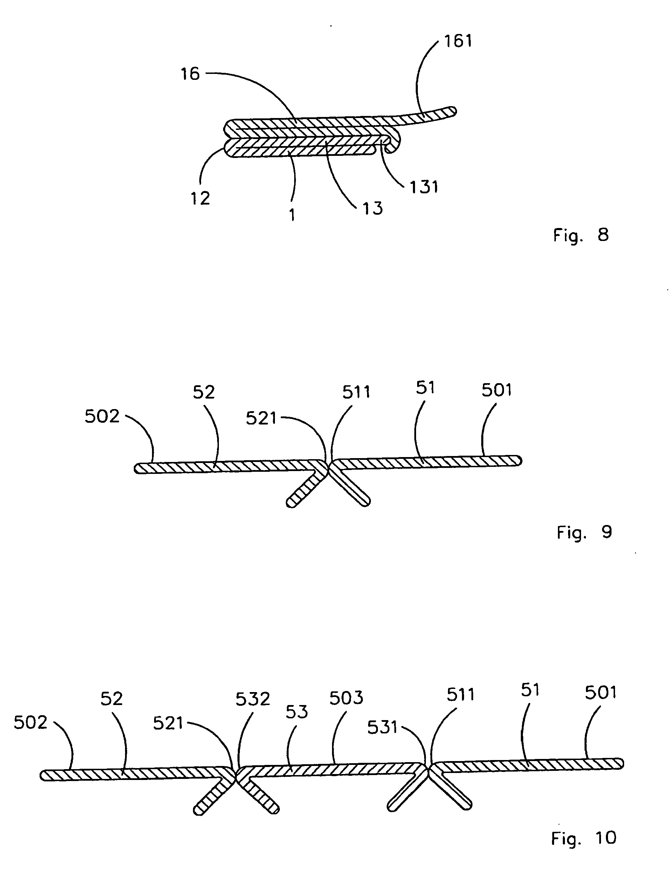

It is to be noted that the first, second and third backing papers are schematically drawn. The arrangement of the said backing papers is discussed on more detail in figure 10.

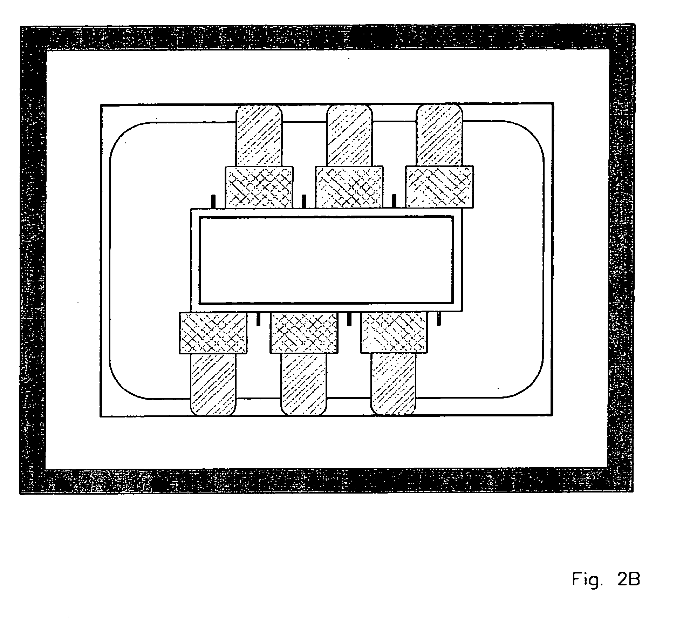

Figure 2B shows the assembly of the separate components of figure 2A. Once applied to the skin of a patient, the device of figure 2 has a two-layered structure, namely the single piece of basic layers, bridging members and spacing members, on the one hand, and the covering sheet on the other.

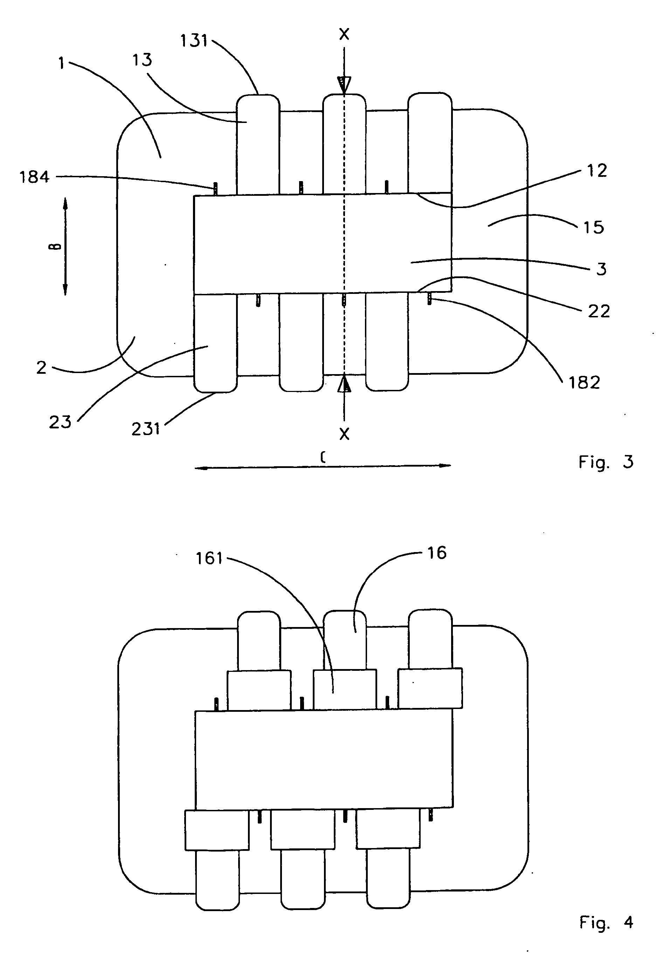

An assembly of first and second sheet members 1, 2, first and second bridging members 13, 23 and spacing members 15 is given in figure 3. Also, alignment markers 184 and 182 are shown. Further, the length of the first opposing edge 12 and second opposing edge 22 is indicated with L. Spacing members 15 define a bridging distance B between the opposing edges 12, 22 of sheet members 1 and 2. Bridging members 13 and 23 are folded back over the opposing edges 12 and 22, respectively, both bridging members having a protruding end, or tip, 131, 231, respectively. The area defined by B and L defines the open skin area 3.

In this view, the second surface of the sheet members and spacing members, as well as the first surface of the bridging members face up.

In figure 4, the assembly as shown in figure 3 is now provided with bridge covering members 16, having a first surface, covering the bridging member there below, and a second surface, facing up. As described in figure 1, the bridge covering member can be connected, via the protruding end 131, 231 of the first and second bridging members 13, 23 to the said bridging members. The exceeding end 161 of the bridge covering member 16 is folded backward at the location of the opposing edges 12, 22 of the sheet members 1, 2 that comprises the said bridging members.

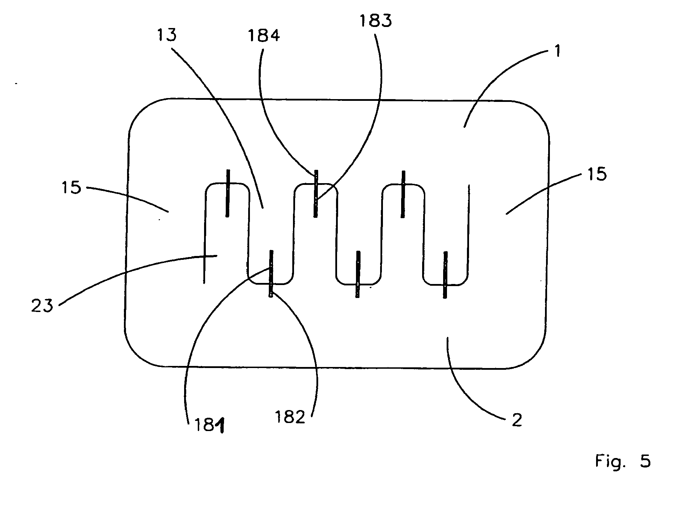

Figure 5 shows the assembly of figure 3 and 4, now in closed position. The first surface of the first and second sheet members, the first and second spacing members 15 and first and second bridging members 13, 23 are faced up down and comprise an adhesive as described above. Bridging member 13 comprises a first alignment marker 181, cooperating with a second alignment marker182 on the second sheet member 2, said second alignment marker 182 being located at the second opposing edge 22, opposite to the location of the first bridging member 13. In this unfolded state, the bridging member extends over the open skin area in the direction of the second sheet member, so that the first and second alignment markers can be aligned to one another when the bridging member is adhered to the skin surrounding the wound. Corresponding third and fourth alignment markers 183 and 184 are present on the second bridging member and the first sheet member, respectively.

It is to be noted that figure 5 shows the shape of the assembly after production thereof. A single sheet of e.g. polyurethane is provided with six lines, forming the first, second, third and fourth alignment markers, each line extending onto a sheet member and a bridging member of the opposing side. Further, the bridging members abut the opposing edge of the opposing sheet member. In this way, the first bridging member extends until the second opposing edge of the second sheet member, and the second bridging member extends until the first opposing edge of the first sheet member.

However, as the sheet material can be stretchable, the bridging members can be stretched upon removing the bridge cover members and pulling the bridging members over the open skin area just before adhering to the skin. By this action, which is further discussed below, the bridging members can be stretched, resulting in extension of the bridging member, so that the protruding ends thereof extend over the opposing sheet members and can be adhered thereon as well. It is however to be noted that a significant portion of the bridging members adhere to the skin, exposed in the open skin area, securing good control of wound closure. By such an extension, the alignment lines 181, 182, and 183, 184, respectively, can be aligned onto one another.

In a very attractive embodiment, as shown in figures 3-5, the bridging members are designed such, that in closed position, i.e. when covering the open skin area in unfolded position, the bridging members lie next to each other and preferably abut one another. More preferably, first and second bridging members abut one another in an alternating fashion. By this arrangement, the complete wound edges will be covered by bridging members, therewith securing an even distribution of the closure forces exerted to the skin, in contrast to the closure by stitching, as discussed above.

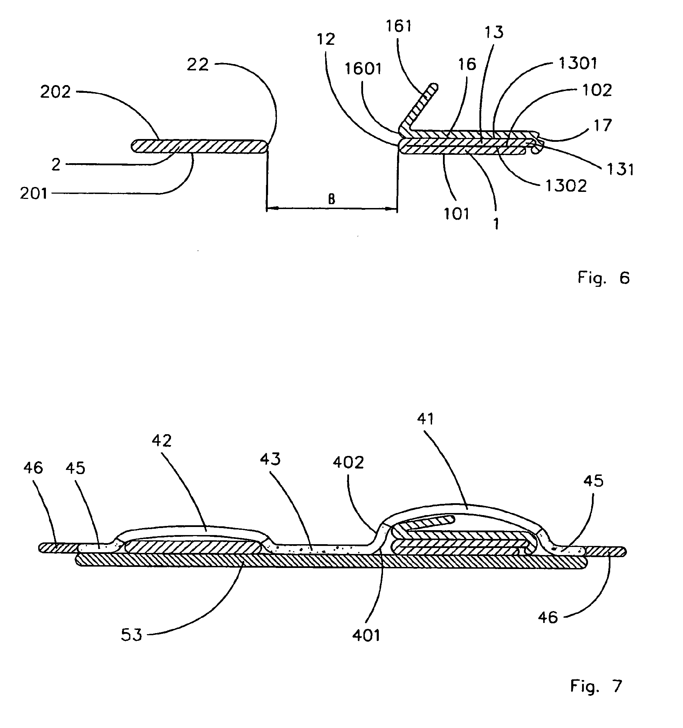

Figure 6 is a cross section at the line, indicated by arrows X in figure 3. It is to be noted that the thickness of the materials indicated are not in scale. At the left, the second sheet member is shown, comprising a first surface 201 and a second surface 202. The first surface 201 comprises an adhesive, for adhering the second sheet member to the skin. Opposed to the second skin member, i.e. at the right in figure 6, sheet member 1 is located at a distance B from the second sheet member, distance B defining the bridging distance between the first and second sheet members at their respective opposing edges 12 and 22. The bridging distance B defines the width of open skin area 3. Sheet member 1 is connected to bridging member 13, folded backward upon sheet member 1. Sheet member 1 has a first surface 101, comprising adhesive for adhering to the skin and a second surface 102, opposed thereto. Bridging member 13 has a protruding end 131 and a first surface 1301 and a second surface 1302, facing to the second surface of the sheet member 1. At the protruding end 131 of bridging member 13, a bridge covering member 16 is connected, the bridge covering member having a first surface 1601, covering the adhering first surface 1301 of bridging member 13. The length of the bridge covering member 16 exceeds that of the bridging member 13. The exceeding end 161 of the bridge covering member 16 is folded backward at the location of the opposing edge 12 of sheet member 1 that comprises bridging member 13. The connection between the bridge covering member 16 and the bridging member 13 comprises a breaking line 17, that allows release of the bridge covering member from the bridging member, when the first surface of the bridge covering member is released from the first surface of the bridging member when a pulling force is exerted to the bridge covering member, e.g. by holding the exceeding end 161 of bridging member 16 by hand, exerting a puling force perpendicular to the device, i.e. in upwards direction in the figure to release the bridge covering member from the first surface of the bridging member, followed by a movement in the direction of the opposing second sheet member. Both movements can also performed simultaneously, as will be shown below.

The cross section shown in figure 7 corresponds to that of figure 6, comprising cover sheet 4 as described for figure 2, however without the shielding member being shown. The assembly comprises a sheet of backing paper 53, and a covering sheet 4. The covering sheet 4 has a first surface 401 facing down, and has a first portion 41, covering the first sheet member 1, a second portion 42, covering the second sheet member 2, and a third portion 43, adhering to the backing paper 53, and circumfering portion 45, also adhering to the backing paper. Circumfering portion 45 of the covering sheet 4 comprises and additional edge portion 46. As explained above, regions 41 and 42 are preferably free of adhesive, or, do at least not adhere to the sheet members 1, 2, and the bridging members 13, 23. To that end, a shielding member can be present (not shown).

In another embodiment, a fourth adhesive can be positioned between the second surface of a sheet member and the second surface of a bridging member, back-folded thereon. In that case, the back-folded position of the bridging member is even more secured when the covering sheet is removed. Such a design makes it even possible to allow regions 41 and 42 of the covering sheet 4 to be adhesive as well, as explained above.

For an even more convenient handling, the bridge covering member has a length, exceeding twice that of the bridging member, so that the end of the bridge covering member 161, extends beyond protruding end 131 of bridging member 13. This is shown in figure 8. At the location of the protruding end 131 of bridge member 13, the bridge covering member is folded backward unto bridging member 13, and, at the location of the opposing edge 12 of sheet member 1, the bridge covering member is folded backward again and as it has a length of more than twice of the bridging member, it has a protruding end 161, extending over the protruding end of the bridging member allowing an easy pick-up by hand.

Figure 9 is a cross section of a first sheet of backing paper 51 and a second sheet of backing paper 52. The first sheet 51 has an edge 511, abutting the second sheet of backing paper at its second abutting edge 521. Both sheets extend beyond their respective abutting edges in order to facilitate manual access to and holding of the sheets. Upper surface 501 of the first sheet of the backing paper and upper surface 502 of the second sheet 52 of backing paper will be in contact with the device according to the present invention, i.e. the first surface of the sheet members and the covering sheet, and in present of the spacing members, which has been explained above. Preferably, these layers are non-adhesive and are hold to the sheet members and the covering sheet by the adhesive surfaces of the said sheet member and covering sheet.

In figure 10, the corresponding situation is shown for three sheets of backing paper. Now, the first sheet 51 abuts via its first abutting edge 511, the third sheet (53) of backing paper at the primary third abutting edge (531) thereof. Accordingly, the third sheet (53) abuts via its secondary third abutting edge (532). The second sheet adds the second abutting edge 521 thereof. Again, all sheets extend beyond there respective abutting edges to facilitate manual handling. Upper surfaces 501, 502 and 503 of the first, second and third sheets, respectively, are to be contacted or are in contact with, the device according to the invention.

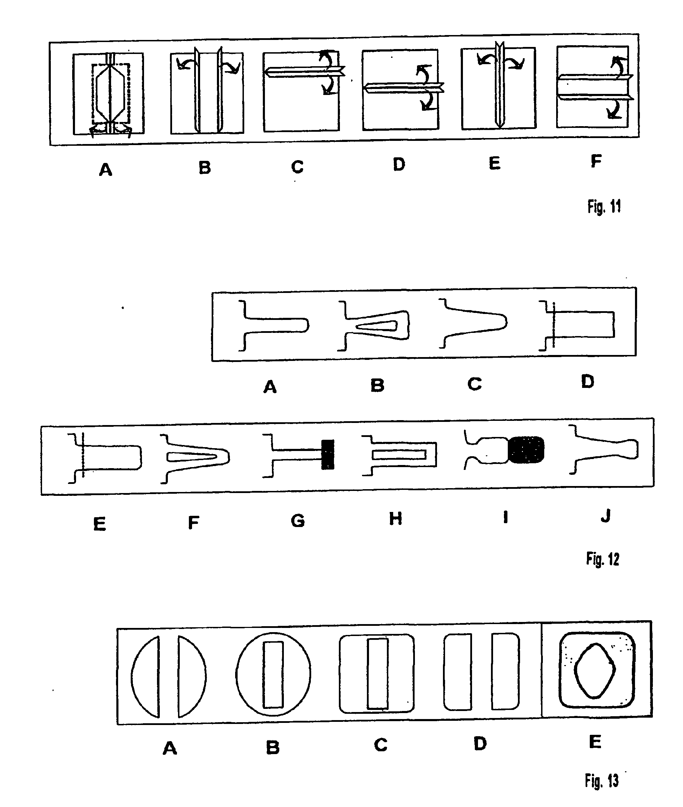

Figure 11 shows a number of alternative embodiments for the shape of the backing paper. In figure 11A, there are two backing papers having abutting edges running in longitudinal direction, i.e. parallel to the opposing edges of the first and second sheet members. As indicated above, multiple sheets of backing material allow proper adherence of the sheet members to the covering sheet therewith avoiding undesired re-positioning of the sheet members upon removal of any of the backing papers. In figure 11B. three sheets of backing papers are shown, having longitudinal abutting edges. The embodiment of figure 11C comprises two sheets of backing paper having abutting edges, running perpendicular to the sheet members, wherein the upper first sheet member is smaller than the lower second sheet member. In this embodiment, the smaller sheet of backing paper is intended to be removed first and the thus exposed portion of the device is adhered to the skin where after the larger sheet of backing paper is removed as well. In this embodiment, it is preferable that the portion of the device, exposed when the smaller sheet of backing paper is removed, adheres to the covering sheet in order to secure proper positioning of e.g. the sheet members of the device. However, although it is preferable, it is not mandatory. The arrows indicate the direction of removal of the respective backing paper from the assembly.

Figure 11D corresponds to the embodiment of figure 11C, however, there are two sheets of backing paper of identical size.

The embodiment of figure 11E corresponds to that of figure 11A, although in figure 11A, portions of the sheets of backing paper extend more beyond the respective abutting edges than in figure 11E.

Figure 11F corresponds with the preferred embodiment, already explained in figure 1**.**

Figure 12. shows different embodiments of the shape of bridging members according to figure 12E is preferred. However, in particular bridging members the shape according to figures 12A, 12C and 12F can be suitable as well. The bridging members of figure 12G and 12I comprise re-enforced members, suitable to be held by hand in order to facilitate handling and unfolding of the bridging members.

In figure 13, several embodiments of the sheet members are given. The embodiments of figures B, C and E comprises pacing members, whereas the embodiments of figures A and D do not. The opposing edges of the first and second sheet members run parallel to one another in the embodiments of figures A-D, but are curved in the embodiment of figure 13E. The embodiment of figure 13C is preferred.

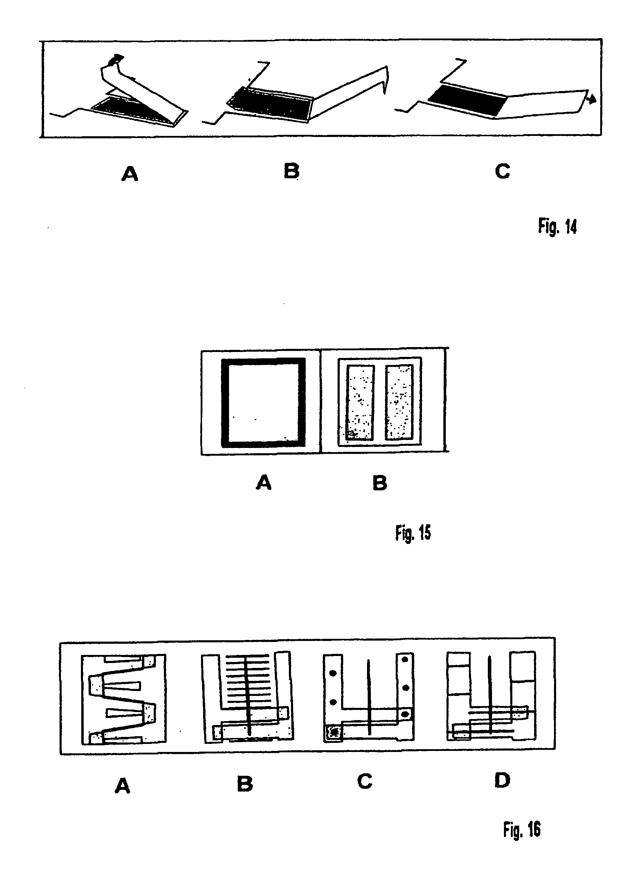

In figure 14, the removal of the bridge covering member, in this case silicone backing paper (however, any suitable material know to the skilled person can be used) is shown. In figure 14A, the backing paper is lifted and the contact with the adhering first surface of the bridging member is obviated. Full exposure of the adhering first surface of the bridging member is shown in figure B. The above discussed braking line is seen in figure 14C. The second adhesive, present on the first surface of the bridging member, is indicated in grey.

In figure 15, two embodiments of covering sheets are shown. In figure 15A, the complete first surface of the covering sheet is provided with an adhesive, and the edges thereof are extended with a stronger sheet like material to improve handling of the device and removal of the covering sheet. In figure 15B, the grey areas are void of adhesive, as these areas will be contacted with the first and second sheet members and bridging members. As discussed above, it is preferable that the covering sheet does not adhere to these portions of the device.

In figure 16, several examples of the alignment markers are shown. In figure 16A, the alignment marker is intrinsically present in the shape of the bridging member. If the bridging members are aligned to one another, a proper positioning of the bridging members is given. In the embodiment of figure 16B, the third portion of the covering sheet comprises a marking dye that marks the skin upon contacting the covering member with the skin. The bridging members can be guided along the markings.

The bridging members can, at their protruding ends, comprise a marking element, such as a dot, as shown in figure 16C, and a corresponding dot can be present near the opposing edge of the opposing sheet member. The markings can have any shape, and it is also possible that the bridging member has one half of a marking, or as the other half is present on the sheet member. However, this embodiment requires relatively long bridging members which can easily be provided with sheet members according to e.g. figures 13A and 13D, and as explained in figure 1. However, in case the sheet members are made of one piece of sheet material, the bridging members have to be stretched to comply with this embodiment.

The embodiment according to figure 16D is preferred and comprises alignment lines as discussed in figures 3-5.

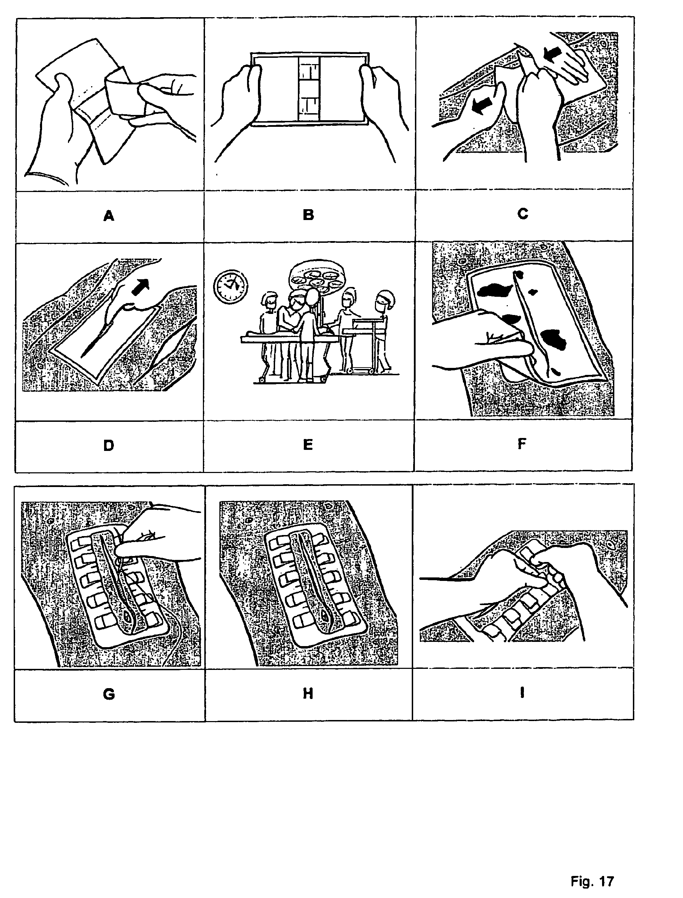

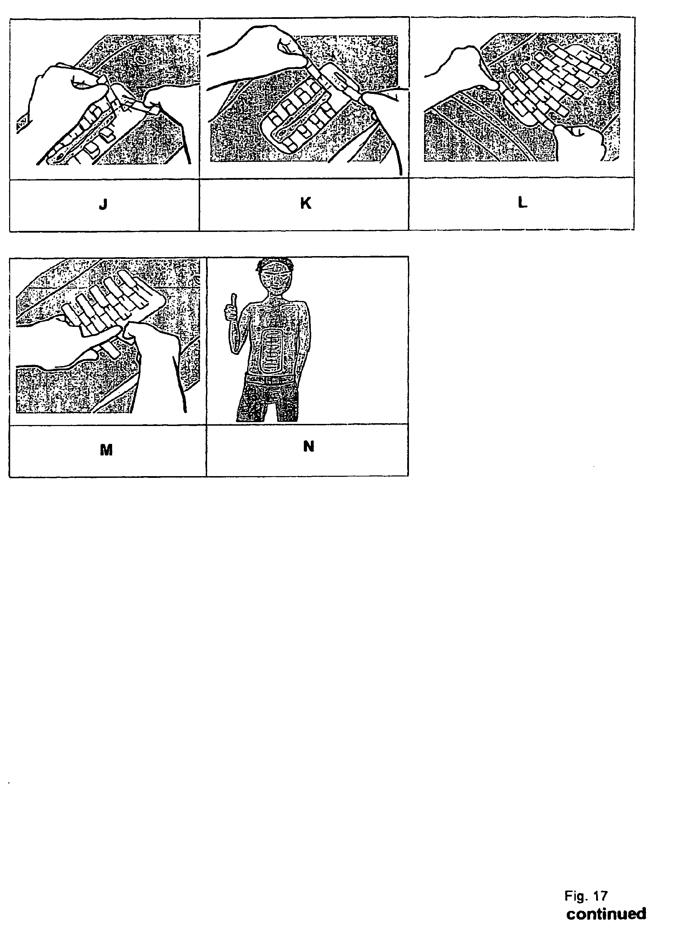

The use of the device is described in figure 17. In figure 17, the device comprises three sheets of backing paper and is of the type as depicted in figure 2. Usually, the device according to the invention is sterilely packed in a suitable bag, or any other suitable packaging means. After removal of the packaging, the surgeon or medical assistant removes the middle (third) part of the backing paper (figure 17A and B) and contacts the exposed portion of the device to the skin of a patient, followed by removal of the first sheet of backing paper, adhering the therewith exposed portion of the device to the skin, and eventually removing the second sheet of backing paper and adhering the therewith exposed portion of the device to the skin, see figure 17C. It is to be observed that the patient is preferably human, but the device may also be applicable on animal skin. Then, the surgeon is allowed to make the incision or excision in the skin, in the present case in the abdomen of a human patient. The device is particularly suitable for use on a human, i.e. for human skin. The incision or excision is made through the third portion of the covering sheet, in the open skin area of the device. After the incision is made, the surgeon can operate the patient, see figure 17E. It is to be noted that in contract to the device, known from the art, not only incisions, but also excisions can be made using the device of the present invention. With the devices according to the state of the art, excisions can not be made, as closure of the wound occurs through re-abutting of the device layer, still sticking to the wound surrounding. Abutting of these layers will not result in wound closure. After the operation, the covering sheet (also named "incision foil") is removed, therewith exposing the wound and surrounding skin in the open skin area, shown in figure 17G. By removal of the covering sheet, the bridging members are exposed. If necessary a subcutaneous suture can be made. It can be clearly seen that the incision or excision wound is now ready for closure, see figure 17H. In figure 17i, the exceeding ends of bridge covering members of two adjacent bridging members are hold by hand and pulled upwards, see figure 17J, so that the first surface of the bridging member is released from the bridge covering member, which bridge covering member is still connected to the tip of the bridging member. Note the presence of the alignment markers on both the bridging member and the sheet members. The corresponding alignment markers are aligned to one another and the bridging members are adhered to the skin, therewith closing the wound, covered by the respective bridging member. Note that the bridging members lie adjacent to one another, and preferably abut one another, see figures 17K and L. As the bridging members are preferably closed in a pair wise fashion, as shown in figures 17F-L, the device comprises preferably the same number of first and second bridging members, arranged in an alternating fashion.

After positioning of the bridging members, the bridge covering members can be removed, e.g. by exerting additional pulling force, allowing release of the bridge covering member by breaking off from the bridging member, e.g. with the aid of the presence of a breaking line, as discussed above (see figure 17M). The device can also be designed such, that a breaking line, as discussed above, is present, which has a tensile strength that is lower than that of the bridging member and of the bridge covering member, so that the bridge covering member breaks from the bridging member before significant deformation of the bridge covering member and/or bridging member can occur. However, some deformation, e.g. stretching as explained above, can be tolerated, as long as the wound closure is secured. The bridge covering members can also be removed during back-folding the bridging members over the wound, i.e. at the moment, depicted in figure 17K. The end result of the closed wound is shown in figure 17N.

The invention encompasses devices for closure an incision in the skin of a patient, as are discussed in the introduction. Alignment markers have not yet been used in devices for closing an incision wound, wherein bridging members are used to bridge the wound and adhere to the surrounding skin. Such devices can have all advantages and features as discussed above, but can also comprise a single sheet member, having two portions, which two portions comprises the bridging members, e.g. as described in WO88/08690. Referring to figure 1 thereof, reference number 2 indicates such a sheet member, on which bridging members 6 are mounted. The portion supporting the left bridging member can be regarded as the first sheet member portion, whereas the right bridging member can be regarded as the second sheet member portion. The incision can be made there between. On the other hand, the sheet member portions can be present as separate from one another, e.g. as shown in figure 1 showing the above-discussed first embodiment by reference numbers 1 and 2 respectively. Devices that are adhered to the skin of a patient where through the incision is to be made, or devices that are adhered to the skin adjacent to the incision once the incision is made, or before the incision is made, such as of the type e.g. shown in US2004/0204740. Therein, sheet member portions 1a and 1 b are separate from one another and each comprise bridging members 5a and 5b, adhering to the opposite sheet member portion. The feature in this example can be seen in the presence of alignment markers, that help to position the bridging members properly as discussed above.

## Claims

1. Device for adhering to the skin of a patient, suitable to make a skin incision there through, the device comprising means for closure of the incision wound and an assembly of:
- a first and a second sheet member (1, 2), each having a first surface (101, 201) and a second surface (102, 202), the second surface being opposed to the first surface,
- the first surface comprising a first adhesive, suitable to adhere the respective sheet member to the skin,
- the first and second sheet members being positioned opposite to one another, the first sheet member having a first opposing edge (12) of a first length (L1), the second sheet member having a second opposing edge (22) of a second length (L2), the first and second opposing edges opposing one another, defining a bridging distance (B) there between,
- the bridging distance and the first and second length of the first and second opposing edges of the first and second sheet members defining an open skin access area (3) to include the wound and surrounding skin therein,
- the first and second sheet members comprising, at their first and second opposing edges respectively, at least one bridging member (13, 23), extending away from the said respective edges and ending in a protruding end (131, 231), the bridging member comprising a strip of sheet material, having a first surface (1301, 2301) and a second surface (1302, 2302), the first surface being opposed to the second surface,
- the at least one bridging member at the first and second sheet member being folded backward onto the first and second sheet members respectively, so that at least a portion of the second surface of the bridging member faces the second surface of the respective sheet member,
- a covering sheet (4), comprising a first surface (401) and a second surface (402), the first surface facing the second surface of the first and second sheet members and the open skin area, the covering sheet comprising a first portion (41), covering the first sheet member, a second portion (42), covering the second sheet member, and a third portion (43), covering the open skin area,
**characterised in that** the first surface of the strip of sheet material of the loridging member comprises a second adhesive, suitable to adhere the respective bridging member to the skin, and optionally also to the second surface of the opposing sheet member, and **in that** the first surface of the covering sheet comprising at the third portion thereof, a third adhesive, suitable to be releasably adhered to the skin.

2. Device according to claim 1, wherein the covering sheet comprises a circumfering portion (45), circumfering the first, second and third portions of the covering sheet, and wherein the first surface of the covering sheet comprises a fourth adhesive at the circumfering portion, which adhesive is suitable to be releasably adhered to the skin
wherein preferably the first surface of the covering sheet covering the first and second sheet members are free of adhesive, and
wherein preferably, the first and second bridging members are an integral part of the first sheet member and the second sheet member respectively.

3. Device according to any of the preceding claims, wherein the first bridging member extends until the second opposing edge of the second sheet member, and the second bridging member extends until the first opposing edge of the first sheet member.

4. Device according to any of the preceding claims, the assembly further comprising a spacing member (15), connected to both the first and the second sheet members, defining the bridging distance there between,
wherein preferably the spacing member is of sheet material, having a first and second surface, the second surface being opposed to the first, the first comprising an adhesive, suitable for releasably adhering the spacing member to the skin, and
the assembly preferably comprising two spacing members defining, together with the first and second opposing edges, the open skin area,
wherein the first and second sheet members and the spacing member (s) are preferably made of a single piece of sheet material.

5. Device according to any of the preceding claims, wherein the covering sheet comprises first visual indication means (47), indicating the location of the first and second sheet member (1, 2), and, if present, the location of the spacing member(s) (15).

6. Device according to any of the preceding claims, wherein the covering sheet comprises second visual indication means (48), indicating the location of the open skin access area (3).

7. Device according to any of the preceding claims, wherein the first surface of each of the bridging members are covered by a first surface (1601) of a bridging covering member (16), so as to cover the adhesive first surface of the bridging members, the first surface of the bridge covering members being releasably attached to the first surface of the respective bridging members;
wherein the bridge covering member is connected to the protruding end of the respective bridging member, and folded backward, onto the first surface thereof.

8. Device according to claim 7, wherein the connection between the bridge covering member and the bridging member comprises a breaking line (17), having a tensile strength being lower than that of the bridging member, and of the bridge covering member, allowing release of the bridge covering member from the bridging member by exerting a pulling force on the bridge covering member.

9. Device according to claim 7 or 8, wherein the length of the bridge covering member exceeds that of the respective bridging member, the exceeding end (161) of the bridge covering member being folded backward at the location of the opposing edge of the sheet member that comprises the said bridging member,
wherein the length of the bridge covering member preferably exceeds twice that of the respective bridging member, so that the exceeding end of the bridge covering member extends beyond the end of the bridging members.

10. Device to any of the preceding claims, wherein the first bridging member comprises a first alignment marker (181) at the protruding end thereof, and the second sheet member comprises a second alignment marker (182) at the second opposing edge, opposite to the location of the first bridging member, so that, upon unfolding the bridging member over the open skin area in the direction of the second sheet member, the first and second alignment markers can be aligned to one another,
wherein preferably the second bridging member comprises a third alignment marker (183) at the protruding end thereof, and the first sheet member comprises a fourth alignment (184) marker at the first opposing edge, opposite to the location of the second bridging member, so that, upon unfolding the bridging member over the open skin area in the direction of the first sheet member, the third and fourth alignment markers can be aligned to one another.

11. Device according to any of the preceding claims, the assembly further comprising a first and a second sheet of backing material (51, 52), the first sheet and second sheet abutting one another via a first abutting edge (511) and a second abutting edge (521) of the first and second sheet of backing paper respectively, wherein preferably the first and second abutting edges are perpendicular to the first and second opposing edges of the first and second sheet members.

12. Device according to any of the preceding claims, the assembly further comprising a first, a second and a third sheet of backing material (51, 52, 53), the third sheet (53) comprising a primary abutting edge (531) and a secondary abutting edge (532), opposed to one another, the third sheet and the first sheet abutting one another via a first abutting edge (511) of the first sheet and the primary abutting edge of the third sheet the third sheet and the second sheet abutting one another via a second abutting edge (521) of the second sheet and the secondary abutting edge of the third sheet,
wherein the first and second abutting edges are perpendicular to the first and second opposing edges of the first and second sheet members

13. Device according to claim 11 or 12, wherein the first and second abutting edges cross the open skin area.

## Patentansprüche

1. Vorrichtung zum Aufkleben auf die Haut eines Patienten, die sich dazu eignet, einen Hauteinschnitt durch sie hindurch zu machen, wobei die Vorrichtung Mittel zum Verschließen der Einschnittwunde und eine Anordnung umfasst aus:
- einem ersten und zweiten Flächenkörperelement (1, 2) mit jeweils einer ersten Fläche (101, 201) und einer zweiten Fläche (102, 202), wobei die zweite Fläche der ersten Fläche entgegengesetzt ist,
- wobei die erste Fläche einen ersten Klebstoff umfasst, der sich dazu eignet, das jeweilige Flächenkörperelement auf die Haut aufzukleben,
- wobei das erste und zweite Flächenkörperelement einander entgegengesetzt angeordnet sind, wobei das erste Flächenkörperelement einen ersten entgegengesetzten Rand (12) mit einer ersten Länge (L1) hat, wobei das zweite Flächenkörperelement einen zweiten entgegengesetzten Rand (22) mit einer zweiten Länge (L2) hat, wobei der erste und zweite entgegengesetzte Rand einander entgegengesetzt sind, wodurch ein Überbrückungsabstand (B) dazwischen definiert ist,
- wobei der Überbrückungsabstand und die erste und zweite Länge des ersten und zweiten entgegengesetzten Rands des ersten und zweiten Flächenkörperelements einen offenen Hautzugangsbereich (3) definieren, der die Wunde und umgebende Haut darin einschließen soll,
- wobei das erste und zweite Flächenkörperelement an ihren ersten bzw. zweiten entgegengesetzten Rändern mindestens ein Überbrückungsteil (13, 23) umfassen, das sich von den jeweiligen Rändern weg erstreckt und in einem vorstehenden Ende (131, 231) endet, wobei das Überbrückungsteil einen Streifen Flächenkörpermaterial mit einer ersten Fläche (1301, 2301) und einer zweiten Fläche (1302, 2302) umfasst, wobei die erste Fläche der zweiten Fläche entgegengesetzt ist,
- wobei das Überbrückungsteil des ersten und zweiten Flächenkörperelement so nach hinten auf das mindestens eine erste bzw. zweite Flächenkörperelement rezurückgeklappt ist, dass mindestens ein Abschnitt der zweiten Fläche des Überbrückungsteils der zweiten Fläche des jeweiligen Flächenkörperelements zugewandt ist,
- einem Abdeckungsflächenkörper (4), der eine erste Fläche (401) und eine zweite Fläche (402) umfasst, wobei die erste Fläche der zweiten Fläche des ersten und zweiten Flächenkörperelements und dem offenen Hautbereich zugewandt ist, wobei der Abdeckungsflächenkörper einen ersten Abschnitt (41), der das erste Flächenkörperelement abdeckt, einen zweiten Abschnitt (42), der das zweite Flächenkörperelement abdeckt, und einen dritten Abschnitt (43) umfasst, der den offenen Hautbereich abdeckt, **dadurch gekennzeichnet, dass** die erste Fläche des Streifens Flächenkörpermaterial des Überbrückungsteils einen zweiten Klebstoff umfasst, der sich dazu eignet, das jeweilige Überbrückungsteil auf die Haut und wahlweise auch auf die zweite Fläche des entgegengesetzten Flächenkörperelements aufzukleben, und dass die erste Fläche des Abdeckungsflächenkörpers an dessen dritten Abschnitt einen dritten Klebstoff umfasst, der sich dazu eignet, lösbar auf die Haut aufgeklebt zu werden.

2. Vorrichtung nach Anspruch 1, wobei der Abdeckungsflächenkörper einen rings umfänglichen Abschnitt (45) umfasst, der den ersten, zweiten und dritten Abschnitt des Abdeckungsflächenkörpers umfänglich umgibt, und wobei die erste Fläche des Abdeckungsflächenkörpers einen vierten Klebstoff am umfänglichen Abschnitt umfasst, welcher Klebstoff dazu geeignet ist, lösbar auf die Haut aufgeklebt zu werden, wobei vorzugsweise die erste Fläche des Abdeckungsflächenkörpers, der das erste und zweite Flächenkörperelement abdeckt, frei von Klebstoff ist, und wobei vorzugsweise das erste und zweite Überbrückungsteil integraler Bestandteil des ersten Flächenkörperelements bzw. zweiten Flächenkörperelements sind.

3. Vorrichtung nach einem der vorhergehenden Ansprüche, wobei sich das erste Überbrückungsteil bis zum zweiten entgegengesetzten Rand des zweiten Flächenkörperelements erstreckt, und wobei sich das zweite Überbrückungsteil bis zum ersten entgegengesetzten Rand des ersten Flächenkörperelements erstreckt.

4. Vorrichtung nach einem der vorhergehenden Ansprüche, wobei die Anordnung darüber hinaus ein Abstandhalterteil (15) umfasst, das sowohl mit dem ersten als auch zweiten Flächenkörperelement verbunden ist und den Überbrückungsabstand zwischen diesen definiert, wobei vorzugsweise das Abstandhalterteil aus Flächenkörpermaterial mit einer ersten und zweiten Fläche besteht, wobei die zweite Fläche der ersten Fläche entgegengesetzt ist, wobei die erste Fläche einen Klebstoff umfasst, der sich dazu eignet, das Abstandhalterteil lösbar auf die Haut aufzukleben, und wobei die Anordnung vorzugsweise zwei Abstandhalterteile umfasst, die zusammen mit dem ersten und zweiten entgegengesetzten Rand den offenen Hautbereich definieren, wobei das erste und zweite Flächenkörperelement und das/die Abstandhalterteil/e vorzugsweise aus einem einzigen Stück Flächenkörpermaterial hergestellt sind.

5. Vorrichtung nach einem der vorhergehenden Ansprüche, wobei der Abdeckungsflächenkörper erste visuelle Anzeigeeinrichtungen (47) umfasst, die die örtliche Lage des ersten und zweiten Flächenkörpers (1, 2) und, falls vorhanden, die örtliche Lage des Abstandhalterteils/der Abstandhalterteile (15) anzeigen.

6. Vorrichtung nach einem der vorhergehenden Ansprüche, wobei das Abdeckungsflächenelement zweite visuelle Anzeigeeinrichtungen (48) umfasst, die die örtliche Lage des offenen Hautzugangsbereichs (3) anzeigen.

7. Vorrichtung nach einem der vorhergehenden Ansprüche, wobei die erste Fläche jedes der Überbrückungsteile durch eine erste Fläche (1601) eines Überbrückungsabdeckungsteils (16) abgedeckt ist, um die klebende erste Fläche der Überbrückungsteile abzudecken, wobei die erste Fläche der Überbrückungsabdeckungsteile lösbar an der ersten Fläche der jeweiligen Überbrückungsteile angebracht ist, wobei das Überbrückungsabdeckungsteil mit dem vorstehenden Ende des jeweiligen Überbrückungsteils verbunden und nach hinten auf dessen erste Fläche zurückgeklappt ist.

8. Vorrichtung nach Anspruch 7, wobei die Verbindung zwischen dem Überbrückungsabdeckungsteil und dem Überbrückungsteil eine Aufreißlinie (17) mit einer Reißfestigkeit umfasst, die geringer ist als diejenige des Überbrückungsteils und des Überbrückungsabdeckungsteils und ein Ablösen des Überbrückungsabdeckungsteils vom Überbrückungsteil ermöglicht, indem eine Zugkraft auf das Überbrückungsabdeckungsteil ausgeübt wird.

9. Vorrichtung nach Anspruch 7 oder 8, wobei die Länge des Überbrückungsabdeckungsteils diejenige des jeweiligen Überbrückungsteils überschreitet, wobei das überschreitende Ende (161) des Überbrückungsabdeckungsteils an der Stelle des entgegengesetzten Endes des Flächenkörperelements nach hinten zurückgeklappt ist, die das Überbrückungsteil umfasst, wobei die Länge des Überbrückungsabdeckungsteils diejenige des jeweiligen Überbrückungsteils vorzugsweise doppelt überschreitet, so dass sich das überschreitende Ende des Überbrückungsabdeckungsteils über das Ende des Überbrückungsteils hinaus erstreckt.

10. Vorrichtung nach einem der vorhergehenden Ansprüche, wobei das erste Überbrückungsteil eine erste Ausrichtungsmarkierung (181) an seinem vorstehenden Ende umfasst, und das zweite Flächenkörperelement eine zweite Ausrichtungsmarkierung (182) am zweiten entgegengesetzten Rand gegenüber der Stelle des ersten Überbrückungsteils umfasst, so dass beim Aufklappen des Überbrückungsteils über dem offenen Hautbereich in Richtung auf das zweite Flächenkörperelement die erste und zweite Ausrichtungsmarkierung miteinander ausgerichtet werden können, wobei vorzugsweise das zweite Überbrückungsteil eine dritte Ausrichtungsmarkierung (183) an seinem vorstehenden Ende umfasst, und das erste Flächenkörperelement eine vierte Ausrichtungsmarkierung (184) am ersten entgegengesetzten Rand gegenüber der Stelle des zweiten Überbrückungsteils umfasst, so dass beim Aufklappen des Überbrückungsteils über dem offenen Hautbereich in Richtung auf das erste Flächenkörperelement die dritte und vierte Ausrichtungsmarkierung miteinander ausgerichtet werden können.

11. Vorrichtung nach einem der vorhergehenden Ansprüche, wobei die Anordnung darüber hinaus eine erste und zweite Schicht Trägermaterial (51, 52) umfasst, wobei die erste Schicht und zweite Schicht über eine erste Stoßkante (511) bzw. zweite Stoßkante (521) der ersten und zweiten Schicht Trägerpapier aneinander anstoßen, wobei vorzugsweise die erste und zweite Stoßkante senkrecht zum ersten und zweiten entgegengesetzten Rand des ersten und zweiten Flächenkörperelements sind.

12. Vorrichtung nach einem der vorhergehenden Ansprüche, wobei die Anordnung darüber hinaus eine erste, eine zweite und eine dritte Schicht Trägermaterial (51, 52, 53) umfasst, wobei die dritte Schicht (53) eine primäre Stoßkante (531) und eine sekundäre Stoßkante (532) umfasst, die einander entgegengesetzt sind, wobei die dritte Schicht und die erste Schicht über eine erste Stoßkante (511) der ersten Schicht und die primäre Stoßkante der dritten Schicht aneinander anstoßen, wobei die dritte Schicht und die zweite Schicht über eine zweite Stoßkante (521) der zweiten Schicht und die sekundäre Stoßkante der dritten Schicht aneinander anstoßen, wobei vorzugsweise die erste und zweite Stoßkante senkrecht zum ersten und zweiten entgegengesetzten Rand des ersten und zweiten Flächenkörperelements sind.

13. Vorrichtung nach Anspruch 11 oder 12, wobei die erste und zweite Stoßkante den offenen Hautbereich überqueren.

## Revendications

1. Dispositif conçu pour adhérer à la peau d'un patient, approprié pour pratiquer une incision cutanée à travers ce dernier, le dispositif comprenant des moyens pour la fermeture de la plaie de l'incision et un ensemble composé de:
- un premier et un deuxième élément de feuille (1, 2), chacun ayant une première surface (101, 201) et une deuxième surface (102, 202), la deuxième surface étant opposée à la première surface,
o la première surface comprenant un premier adhésif approprié pour faire adhérer l'élément de feuille respectif à la peau,
o les premier et deuxième éléments de feuille étant positionnés de manière opposée l'un par rapport à l'autre, le premier élément de feuille ayant un premier bord opposé (12) d'une première longueur (L1), le deuxième élément de feuille ayant un deuxième bord opposé (22) d'une deuxième longueur (L2), les premier et deuxième bords opposés étant opposés l'un par rapport à l'autre, définissant une distance de liaison (B) entre eux,
o la distance de liaison et les première et deuxième longueurs des premier et deuxième bords opposés des premier et deuxième éléments de feuille définissant une zone d'accès cutanée ouverte (3) pour inclure la plaie et la peau périphérique à l'intérieur de cette dernière,
- les premier et deuxième éléments de feuille comprenant, au niveau de leurs premier et deuxième bords opposés respectivement, au moins un élément de liaison (13, 23), s'étendant à distance desdits bords respectifs et se terminant par une extrémité en saillie (131, 231), l'élément de liaison comprenant une bande de matériau en feuille, ayant une première surface (1301, 2301) et une deuxième surface (1302, 2302), la première surface étant opposée à la deuxième surface,
- le au moins un élément de liaison du premier et deuxième élément de feuille étant plié vers l'arrière sur le premier et deuxième élément de feuille, respectivement, de sorte qu'au moins une partie de la deuxième surface de l'élément de liaison fait face à la deuxième surface de l'élément de feuille respectif,
- une feuille de recouvrement (4) comprenant une première surface (401) et une deuxième surface (402), la première surface faisant face à la deuxième surface des premier et deuxième éléments de feuille et à la zone cutanée ouverte, la feuille de recouvrement comprenant une première partie (41) recouvrant le premier élément de feuille, une deuxième partie (42) recouvrant le deuxième élément de feuille, et une troisième partie (43) recouvrant la zone cutanée ouverte, **caractérisé en ce que** la première surface de la bande de l'élément de feuille de l'élément de liaison comprend un deuxième adhésif, approprié pour faire adhérer l'élément de liaison respectif à la peau, et facultativement également à la deuxième surface de l'élément de feuille opposé, **et en ce que** la première surface de la feuille de recouvrement comprend au niveau de sa troisième partie, un troisième adhésif approprié pour être fixé de manière amovible sur la peau.

2. Dispositif selon la revendication 1, dans lequel la feuille de recouvrement comprend une partie circonférentielle (45), entourant les première, deuxième et troisième parties de la feuille de recouvrement, et dans lequel la première surface de la feuille de recouvrement comprend un quatrième adhésif au niveau de la partie circonférentielle, lequel adhésif est approprié pour être fixé de manière amovible sur la peau, dans lequel,de préférence la première surface de la feuille de recouvrement recouvrant les premier et deuxième éléments de feuille est dépourvue d'adhésif, et dans lequel de préférence les premier et deuxième éléments de liaison font partie intégrante du premier élément de feuille et du deuxième élément de feuille respectivement.

3. Dispositif selon l'une quelconque des revendications précédentes, dans lequel le premier élément de liaison s'étend jusqu'au deuxième bord opposé du deuxième élément de feuille, et le deuxième élément de liaison s'étend jusqu'au premier bord opposé du premier élément de feuille.

4. Dispositif selon l'une quelconque des revendications précédentes, l'ensemble comprenant en outre un élément d'espacement (15) raccordé à la fois aux premier et deuxième éléments de feuille, définissant la distance de liaison entre eux, dans lequel de préférence l'élément d'espacement est réalisé avec un matériau en feuille, ayant une première et une deuxième surface, la deuxième surface étant opposée à la première, la première comprenant un adhésif approprié pour fixer de manière amovible l'élément d'espacement sur la peau, et l'ensemble, comprenant de préférence deux éléments d'espacement définissant, conjointement avec les premier et deuxième bords opposés, la zone cutanée ouverte, dans lequel les premier et deuxième éléments de feuille et l'élément d'espacement (les éléments d'espacement) sont de préférence réalisés avec un matériau en feuille d'un seul tenant.

5. Dispositif selon l'une quelconque des revendications précédentes, dans lequel la feuille de recouvrement comprend des premiers moyens d'indication visuelle (47), indiquant l'emplacement des premier et deuxième éléments de feuille (1, 2) et s'il existe, l'emplacement du (des) élément(s) d'espacement (15).

6. Dispositif selon l'une quelconque des revendications précédentes, dans lequel la feuille de recouvrement comprend des deuxièmes moyens d'indication visuelle (48), indiquant l'emplacement de la zone d'accès cutanée ouverte (3).

7. Dispositif selon l'une quelconque des revendications précédentes, dans lequel la première surface de chacun des éléments de liaison est recouverte par une première surface (1601) d'un élément de recouvrement de liaison (16), afin de recouvrir la première surface adhésive des éléments de liaison, la première surface des éléments de recouvrement de liaison étant fixée de manière amovible à la première surface des éléments de liaison respectifs, dans lequel l'élément de recouvrement de liaison est raccordé à l'extrémité en saillie de l'élément de liaison respectif, et plié vers l'arrière, sur sa première surface.

8. Dispositif selon la revendication 7, dans lequel le raccordement entre l'élément de recouvrement de liaison et l'élément de liaison comprend une ligne de rupture (17), ayant une résistance à la traction inférieure à celle de l'élément de liaison, et de l'élément de recouvrement de liaison, permettant la libération de l'élément de recouvrement de liaison de l'élément de liaison en exerçant une force de traction sur l'élément de recouvrement de liaison.

9. Dispositif selon la revendication 7 ou 8, dans lequel la longueur de l'élément de recouvrement de liaison est supérieure à celle de l'élément de liaison respectif, l'extrémité en excès (161) de l'élément de recouvrement de liaison étant repliée en arrière à l'emplacement du bord opposé de l'élément de feuille qui comprend ledit élément de liaison, dans lequel la longueur de l'élément de recouvrement de liaison est de préférence deux fois supérieure à celle de l'élément de liaison respectif, de sorte que l'extrémité en excès de l'élément de recouvrement de liaison s'étend au-delà de l'extrémité de l'élément de liaison.

10. Dispositif selon l'une quelconque des revendications précédentes, dans lequel le premier élément de liaison comprend un premier marqueur d'alignement (181) au niveau de son extrémité en saillie, et le deuxième élément de feuille comprend un deuxième marqueur d'alignement (182) au niveau du deuxième bord opposé, opposé à l'emplacement du premier élément de liaison, de sorte que, suite au dépliage de l'élément de liaison sur la zone cutanée ouverte dans la direction du deuxième élément de feuille, les premier et deuxième marqueurs d'alignement peuvent être alignés l'un par rapport à l'autre, dans lequel, de préférence le deuxième élément de liaison comprend un troisième marqueur d'alignement (183) au niveau de son extrémité en saillie, et le premier élément de feuille comprend un quatrième marqueur d'alignement (184) au niveau du premier bord opposé, opposé à l'emplacement du deuxième élément de liaison de sorte que, suite au dépliage de l'élément de liaison sur la zone cutanée ouverte dans la direction du premier élément de feuille, les troisième et quatrième marqueurs d'alignement peuvent être alignés l'un par rapport à l'autre.

11. Dispositif selon l'une quelconque des revendications précédentes, l'ensemble comprenant en outre une première et une deuxième feuille de matériau antiadhésif (51, 52), les première et deuxième feuilles venant en butée l'une contre l'autre via un premier bord de butée (511) et un deuxième bord de butée (521) des première et deuxième feuilles de matériau antiadhésif respectivement, dans lequel de préférence les premier et deuxième bords de butée sont perpendiculaires aux premier et deuxième bords opposés des premier et deuxième éléments de feuille.

12. Dispositif selon l'une quelconque des revendications précédentes, l'ensemble comprenant en outre une première, une deuxième et une troisième feuille de matériau antiadhésif (51, 52, 53), la troisième feuille (53) comprenant un bord de butée principal (531) et un bord de butée secondaire (532) opposés l'un par rapport à l'autre, la troisième feuille et la première feuille venant en butée l'une contre l'autre via un premier bord de butée (511) de la première feuille et le bord de butée principal de la troisième feuille, la troisième feuille et la deuxième feuille venant en butée l'une contre l'autre via un deuxième bord de butée (521) de la deuxième feuille et le bord de butée secondaire de la troisième feuille, dans lequel de préférence les premier et deuxième bords de butée sont perpendiculaires aux premier et deuxième bords opposés des premier et deuxième éléments de feuille.

13. Dispositif selon la revendication 11 ou 12, dans lequel les premier et deuxième bords de butée traversent la zone cutanée ouverte.
